# EUROPÄISCHE PATENTSCHRIFT

(11) **EP 2 948 071 B1**
(45) Veröffentlichungstag und Bekanntmachung des Hinweises auf die Patenterteilung: **22.08.2018**
(21) Anmeldenummer: 14700932.8
(22) Anmeldetag: 20.01.2014
(51) Int. Cl.: A61B 17/132, A61B 17/00, A61B 90/00

(54) **ABSCHNÜRVORRICHTUNG**
CONSTRICTING DEVICE
DISPOSITIF DE CONSTRICTION

(30) Priorität: 23.01.2013 DE 202013100313 U; 21.11.2013 DE 202013112844 U
(43) Veröffentlichungstag der Anmeldung: 02.12.2015
(73) Patentinhaber: Kimetec GmbH, 71254 Ditzingen (DE)
(72) Erfinder: KIRCHNER, Hansjörg, 71706 Markgröningen (DE); KIRCHNER, Claudia, 71706 Markgröningen (DE); IHLE, Caroline, 71706 Markgröningen (DE)
(74) Vertreter: Fleck, Hermann-Josef
(86) Internationale Anmeldenummer: PCT/EP2014/051055
(87) Internationale Veröffentlichungsnummer: WO 2014/114604

(56) Entgegenhaltungen:
- EP-A2- 2 045 047
- WO-A1-2006/015987
- DE-A1-102009 025 416
- GB-A- 479 442
- GB-A- 1 033 130
- US-A1- 2009 043 168

## Beschreibung

Die Erfindung bezieht sich auf einen Venenstauer, mit einem um ein Körperteil legbaren Abschnürband, einer Verschlussvorrichtung, mittels deren das Abschnürband in seinem um ein Körperteil gelegten gespannten Zustand zu einer Schlaufe verriegelbar ist, und mit einem Spannungsindikator zum Feststellen eines Spannungszustandes des Abschnürbandes.

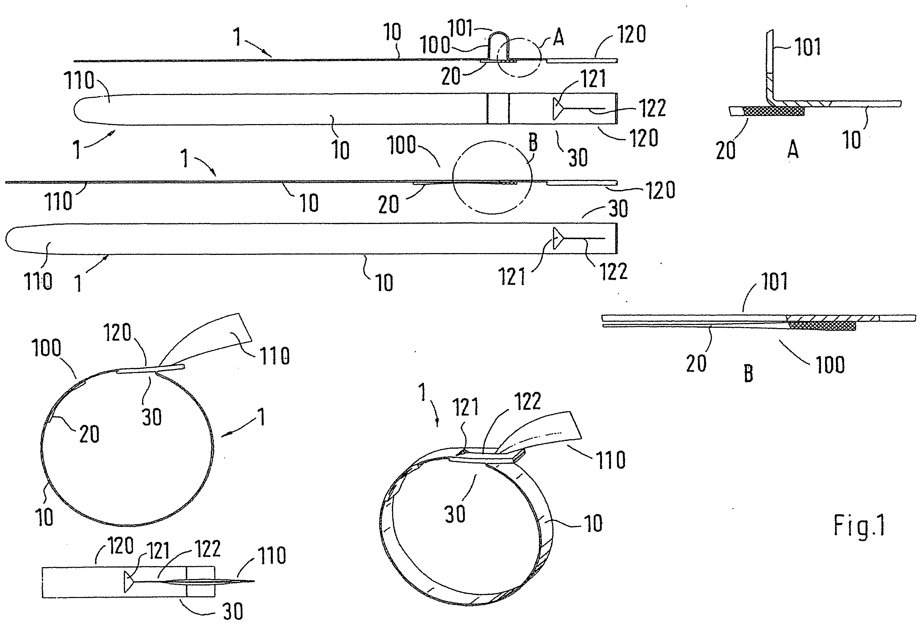

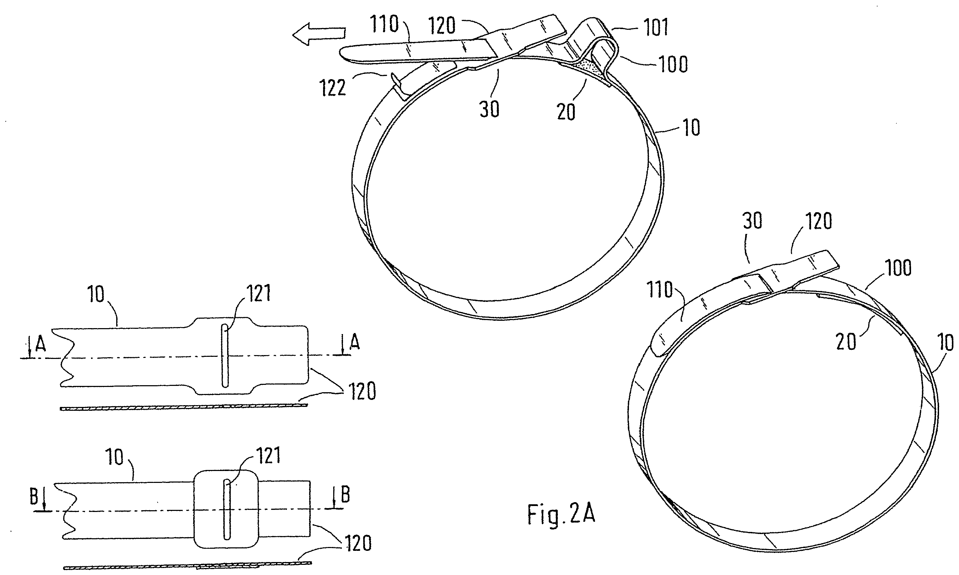

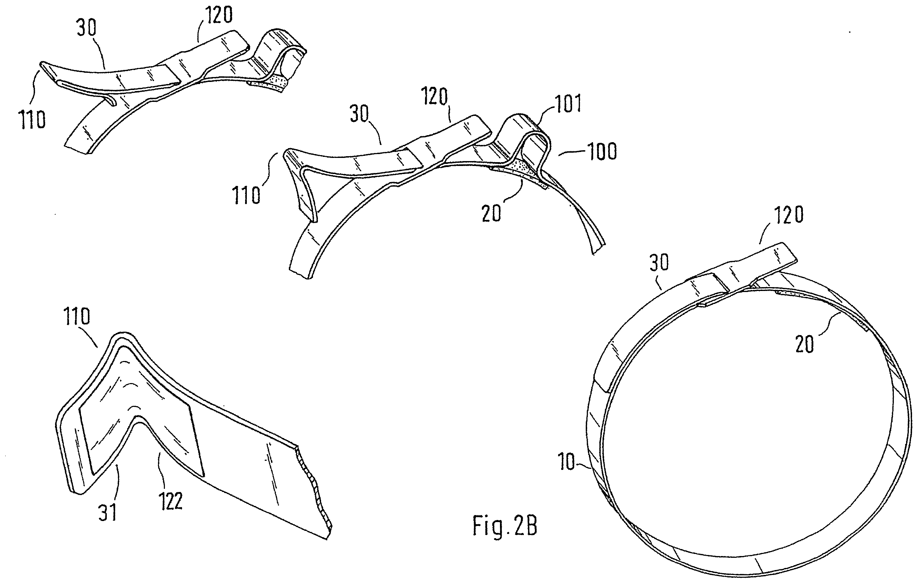

In der DE 10 2009 025416 A1 ist ein Stütz- oder Fixiergurt angegeben, wobei Gurte mit Verschlussvorrichtung in Form eines Klettverschlusses, mit dem der Gurt zu einer Schlaufe verriegelbar ist, offenbart sind. Der Gurt weist einen parallel zu einem unelastischen Abschnitt liegenden elastischen Bereich auf, wobei die dortige Idee zwei Alternativen beinhaltet, nämlich eine, bei der der unelastische Abschnitt kürzer ist als der elastische Abschnitt (Beispiel nach Fig. 1), so dass der Gurt durch Durchtrennen des unelastischen Abschnitts eine Dynamisierung des Gurtes ergibt und dieser zumindest teilweise elastisch ist, und eine andere Alternative, bei der der unelastische Abschnitt länger als der elastische Abschnitt ist, um eine Überlastsicherung des elastischen Bereichs vorzusehen, so dass vermieden werden kann, dass der elastische Abschnitt über die maximale Dehngrenze hinaus verlängert wird, wobei zumindest ein Teil der Zugkraft über die unelastischen Abschnitte weitergeleitet wird (Ausführung nach Fig. 2). Hierbei übernimmt der den elastischen Abschnitt überbrückende Abschnitt also maßgeblich die Zugkraft des Gurtes.

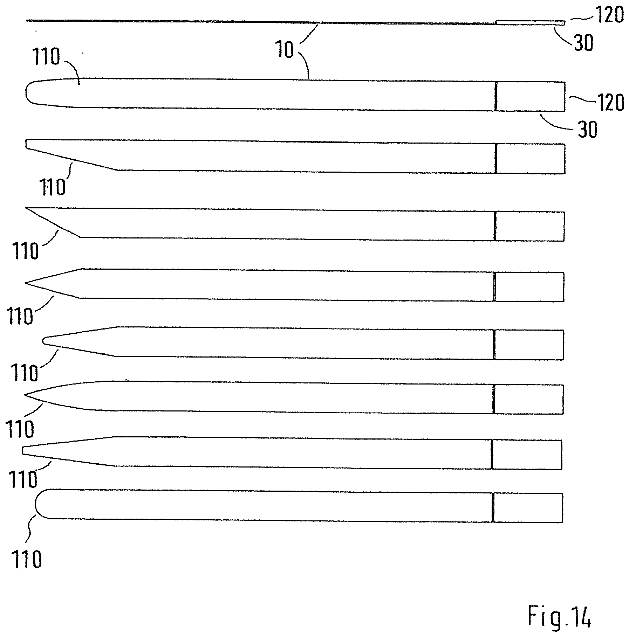

Eine weitere Abschnürvorrichtung ist in der US 2012/0071917 A1 angegeben. Bei dieser bekannten Abschnürvorrichtung, die insbesondere bei Verletzungen zum Unterbinden von starken Blutungen eines Körperteils dient, sind eine Spannvorrichtung zum Erzielen einer hohen Spannung des um das Körperteil zu einer Schlaufe gelegten Abschnürbandes sowie ein Spannungsindikator vorgesehen, mit dem eine erzeugte Spannung des Abschnürbandes taktil oder visuell festgestellt werden kann. Beispielsweise ist hierzu, wie in den dortigen Fig. 14A und 14B mit Beschreibung gezeigt, ein Spannungsgewebe gefaltet und im Faltenbereich an Befestigungsstellen festgelegt, die bei übermäßiger Spannung reißen. Die Befestigungsstellen können über die Breite des Spannungsgewebes unterschiedlich viele Befestigungspunkte aufweisen, wodurch die Befestigungsstellen bei entsprechend unterschiedlicher Zugspannung reißen. Allerdings muss eine derart hohe Zugspannung erst aufgebracht werden, um ein Reißen an der oder den Befestigungsstellen zu bewirken, wonach dann aber die Zugspannung abrupt nachlässt. Mit diesen Maßnahmen ist eine dosierte Spannung, wie sie beispielsweise bei Venenstauern zur Wirkung kommt, um einen venösen Blutfluss zu stauen, schwer erkennbar.

Weitere Abschnürvorrichtungen für Körperteile zeigen die US 5,653,728 A, die WO 2006/015987 A1, die GB 2 424 189 A, die US 3,628,536 A, die US 3,930,506 A, die US 6,525,238 B2, die US 2008/0312682 A1 sowie die US 6,250,047 B1. Dabei sind beispielsweise in der US 5,653,728 A und der WO 2006/015987 A1 auch als nach Gebrauch wegwerfbare Ausführungen von Abschnürvorrichtungen offenbart, wie sie häufig im klinischen Bereich zum Einhalten von Hygienevorschriften benötigt werden. Bei den genannten Druckschriften zum Teil vorhandene Spannungsindikatoren sind relativ kompliziert aufgebaut (s. z.B. US 2008/0312682 A1). Die in der US 6,525,238 B2 gezeigte Vorrichtung bildet eine Zwischenlage zwischen einem Körperteil und einer Blutdruck-Messmanschette ohne einsehbaren Spannungsindikator.

Der Erfindung liegt die Aufgabe zugrunde, einen Venenstauer der eingangs genannten Art bereit zu stellen, mit dem die Benutzung erleichtert wird.

Diese Aufgabe wird mit den Merkmalen des Anspruches 1 gelöst. Hierbei ist vorgesehen, dass der Spannungsindikator als ein einen elastischen Abschnitt am Abschnürband in Dehnrichtung zwischen zwei Fixierstellen mit Überlänge bezüglich des ungedehnten Abschnürbandes überbrückender Bahnabschnitt in der Weise ausgebildet ist, dass ein definierter Spannungszustand von einem Benutzer erkennbar ist.

Mit diesen Maßnahmen kann der Benutzer, beispielsweise bei einer Ausbildung der Abschnürvorrichtung als Venenstauer, die zum Unterbinden des Blutflusses erforderliche, relativ geringe Zugspannung (von z. B. ca. 10 mm Hg im Bereich von Kapillargefäßen oder < 25 mm Hg im Bereich größerer Venen) exakt einstellen. Hierdurch wird auch die behandelte Person nicht mehr als nötig belastet.

Ist vorgesehen, dass der Spannungsindikator auf der im Gebrauchszustand von dem Körperteil abgekehrten Außenseite des Abschnürbandes angeordnet ist, so kann die erzeugte Spannung leicht erkannt und eingestellt werden.

Eine vorteilhafte Ausgestaltung zum Erkennen der erzeugten Zugspannung besteht darin, dass der Spannungsindikator als ein einen elastischen Abschnitt am Abschnürband in Dehnrichtung zwischen zwei Fixierstellen mit definierter Überlänge bezüglich des unbelasteten Abschnürbandes überbrückender Bahnabschnitt aus einem flexiblen (im Wesentlichen, relativ zu dem elastischen Abschnitt) unelastischen Material ausgebildet ist. Ist der mit einer Überlänge bezüglich des Abstandes der Fixierstellen im spannungslosen Zustand des Abschnürbandes (z. B. als gefalteter Abschnitt) ausgeführte Bahnabschnitt noch locker, so ist ein definiert vorgegebener Spannungszustand noch nicht erreicht. Ist hingegen der flexible unelastische Bahnabschnitt infolge des gedehnten elastischen Abschnitts zwischen den Fixierstellen vollkommen entlang dem Abschnürband erstreckt, so ist der definierte Spannungszustand z. B. zum Unterdrücken des venösen Blutflusses erreicht. Durch die Überlänge des Bahnabschnitts zwischen den beiden Fixierstellen kann somit ein gewünschter definierter Spannungszustand für einen jeweiligen Anwendungsfall herstellerseitig vorgegeben werden, der bei der Anwendung von dem Benutzer leicht und eindeutig erkennbar und einstellbar ist.

Eine vorteilhafte Ausgestaltungsvariante besteht dabei darin, dass das Abschnürband aus einem flexiblen unelastischen Material ausgebildet ist und der Bahnabschnitt einen integralen Teil des Abschnürbandes bildet, wobei an den Fixierstellen ein den elastischen Abschnitt bildendes elastisches Element angebunden ist.

Eine andere vorteilhafte Ausgestaltungsvariante besteht darin, dass das Abschnürband selbst zumindest abschnittsweise als elastisches Band ausgebildet ist und der Bahnabschnitt aus unelastischem Material an den Fixierstellen an dem elastischen Band angebunden ist.

Eine für einen Einmalgebrauch vorteilhafte Abschnürvorrichtung besteht darin, dass der elastische Abschnitt oder ein anderer Abschnitt des elastischen Bandes mit einem bei einer ersten Dehnung irreversibel durchreißenden oder beschädigten Gebrauchsindikator versehen ist, wie z B. aus Papier oder Lack. Die Durchreißkraft des Gebrauchsindikators ist hierbei vorteilhaft gering, so dass sie den Spannungsvorgang des Abschnürbandes möglichst wenig beeinflusst und der Durchriss beispielsweise bereits bei geringer Dehnung von wenigen Prozent der Dehnung bei dem definierten Spannungszustand (z. B. nicht mehr als 10 % oder 20 % der Dehnung im definierten Spannungszustand) eintritt. Dies kann durch z. B. entsprechende Materialwahl und/oder Materialstärke und/oder Form des Gebrauchsindikators oder durch Abriss an einer Anbindungsstelle erreicht werden.

Für den Gebrauch und die Funktion der Abschnürvorrichtung sind des Weiteren die Maßnahmen von Vorteil, dass ein Endabschnitt des Abschnürbandes als verschlussseitiger Bandabschnitt mit einer Verschlussöffnung zum Durchführen des anderen endseitigen Bandabschnitts und mit einem Halteteil zum Festhalten des um einen Körperteil gelegten Abschnürbandes im gespannten Zustand versehen ist. Der verschlussseitige Bandabschnitt ist hierbei vorteilhaft als verstärktes Endteil des Abschnürbandes selbst oder als ein daran befestigter verstärkter Abschnitt ausgebildet.

Verschiedene vorteilhafte Ausgestaltungsvarianten bestehen dabei darin, dass das Halteteil als mindestens ein Klemmschlitz, als in eine Lochanordnung des Abschnürbandes eingreifender Haltezapfen, als Klettverschlussabschnitt, als Klebeteil, als Druckknopfteil oder als schwenkbare Klemmplatte ausgebildet ist. Für eine Ausbildung mit schwenkbarer Klemmplatte ist z. B. die Ausgestaltung der Verschlussvorrichtung entsprechend der EP 0 633 747 B1 oder der EP 1 458 296 B1 von Vorteil. Hierbei ist die Klemmplatte in einem an dem verschlussseitigen Bandabschnitt angebrachten Schlossgehäuse angeordnet.

Eine insbesondere für den Einmalgebrauch vorteilhafte Ausgestaltung wird dadurch erhalten, dass das Abschnürband aus reißfestem gewebeartigen, vliesartigen oder papierartigen Material hergestellt ist. Das reißfeste papierartige Material kann dabei z. B. Verstärkungsfasern enthalten und teilweise auch aus Kunststoffmaterial bestehen. Alternativ ist auch die vollständige Herstellung aus Kunststoff möglich.

Für die Fertigung und Funktion sind die Maßnahmen von Vorteil, dass die Anbindung durch Befestigen des elastischen Abschnitts oder des Bahnabschnitts an dem Abschnürband an den Fixierstellen mittels Klebens, Schweißens, thermoplastischer Verfahren oder Vernähens hergestellt ist.

Die Erfindung wird nachfolgend anhand von Ausführungsbeispielen unter Bezugnahme auf die Zeichnungen näher erläutert. Es zeigen:
- Fig. 1: ein erstes Ausführungsbeispiel für eine Abschnürvorrichtung in verschiedenen Darstellungen, nämlich (von oben nach unten) einer Seitenansicht und einer Draufsicht sowie einer Detailansicht im spannungslosen Nichtgebrauchszustand, einer Seitenansicht und Draufsicht sowie Detailansicht im gespannten Zustand einer Seitenansicht und Draufsicht in einem zu einer Schlaufe gelegten Zustand und einer perspektivischen Darstellung in dem zu einer Schlaufe gelegten Zustand,
- Fig. 2A: zwei perspektivische Darstellungen eines weiteren Ausführungsbeispiels der Abschnürvorrichtung mit anderer Verschlussvorrichtung in einem nicht gespannten und einem gespannten, zu einer Schlaufe gelegten Zustand sowie zwei vergrößerte Darstellungen in Draufsicht und Seitenansicht für zwei Varianten eines verschlussseitigen Bandabschnitts,
- Fig. 2B: ein weiteres Ausführungsbeispiel der Abschnürvorrichtung in perspektivischen Darstellungen mit einer anderen Verschlussvorrichtung,
- Fig. 3: ein weiteres Ausführungsbeispiel der Abschnürvorrichtung mit einer anderen Verschlussvorrichtung in einer perspektivischen Darstellung mit dem zu einer Schlaufe gelegten Abschnürband im gespannten Zustand und einem betreffenden endseitigen Bandabschnitt im nicht gespannten Zustand sowie eine Seitenansicht und Draufsicht des endseitigen Abschnitts im nicht gespannten Zustand sowie des endseitigen Bandabschnitts im gespannten Zustand,
- Fig. 4: verschiedene weitere Ausführungsbeispiele für den verschlussseitigen Bandabschnitt von Abschnürvorrichtungen in Seitenansicht und in Draufsicht sowie (zum Teil) zusätzlich in einer perspektivischen Ansicht oder Ansicht von unten,
- Fig. 5: weitere Ausführungsbeispiele für Abschnürvorrichtungen im nicht gespannten Zustand, teilweise auch nur abschnittsweise im Bereich des verschlussseitigen Bandabschnitts in Seitenansicht und Draufsicht sowie einer perspektivischen Darstellung,
- Fig. 6: weitere Ausführungsbeispiele für Abschnürvorrichtungen mit alternativen Ausgestaltungen des verschlussseitigen Bandabschnittes in Seitenansicht, Draufsicht und perspektivischen Ansichten,
- Fig. 7: weitere Ausführungsbeispiele für Abschnürvorrichtungen in Seitenansicht, Draufsicht und perspektivischer Ansicht im Bereich des verschlussseitigen Bandabschnittes,
- Fig. 8: ein weiteres Ausführungsbeispiel für eine Abschnürvorrichtung im Bereich des verschlussseitigen Bandabschnitts in Seitenansicht, Draufsicht und einer perspektivischen Darstellung,
- Fig. 9: weitere Ausführungsbeispiele für Abschnürvorrichtungen mit verschiedenen Lochgestaltungen für die Kopplung mittels der Verschlussvorrichtung in betreffenden Bandabschnitten in Draufsicht,
- Fig. 10: ein weiteres Ausführungsbeispiel der Abschnürvorrichtung im nicht gespannten Zustand des Abschnürbandes und im gespann-ten Zustand desselben in Draufsicht, Seitenansicht, Unteransicht und perspektivischer Ansicht sowie vergrößerte Ausschnittsdarstellungen in den beiden Zuständen,
- Fig. 11: ein weiteres Ausführungsbeispiel für eine Abschnürvorrichtung im nicht gespannten und im gespannten Zustand in Seitenansicht und Draufsicht,
- Fig. 12: ein weiteres Ausführungsbeispiel für eine Abschnürvorrichtung mit einem Gebrauchsindikator in zwei perspektivischen Ansichten sowie in zwei Draufsichtdarstellungen, von denen die eine den nicht gespannten Zustand des Abschnürbandes und die andere einen gespannten Zustand desselben zeigt,
- Fig. 13: verschiedene weitere Ausführungsbeispiele der Abschnürvorrichtung mit betreffenden Bandabschnitten in Draufsicht, die mit verschiedenen Gebrauchsindikatoren versehen sind, jeweils im nicht gespannten und im gespannten Zustand des Abschnürbandes,
- Fig. 14: weitere Ausführungsbeispiele für die Abschnürvorrichtung in einer Seitenansicht und Draufsichten mit verschiedenen endseitigen Bandabschnitten,
- Fig. 15: verschiedene weitere Ausführungsbeispiele für die Abschnürvorrichtung in einer Seitenansicht und einer Draufsicht des verschlussseitigen Bandabschnitts mit unterschiedlichen Verschlussöffnungen der Verschlussvorrichtung und
- Fig. 16: weitere Ausführungsbeispiele der Abschnürvorrichtung in einer perspektivischen Darstellung mit zu einer Schlaufe gelegtem Abschnürband und einem Ausschnitt desselben im Bereich der Verschlussvorrichtung, den verschlussseitigen Bandabschnitt der perspektivischen Darstellung in Draufsicht und zwei weitere Ausführungsbeispiele für die Ausgestaltung des verschlussseitigen Bandabschnitts mit verschiedenen Verschlussöffnungen und als Klemmschlitz ausgebildeten Halteteilen in Draufsicht.

Fig. 1 zeigt ein erstes Ausführungsbeispiel für eine Abschnürvorrichtung 1 mit einem Abschnürband 10, das zum zumindest teilweisen Unterbinden eines Blutflusses in Form einer geschlossenen Schlaufe um einen Körperteil legbar und im gespannten Zustand mittels einer Verschlussvorrichtung 30 fixierbar ist. Die Abschnürvorrichtung 1 weist einen Spannungsindikator 101 auf, mit dem die Spannkraft des Abschnürbandes 10 im Bereich der um einen Körperteil gelegten Schlaufe von einem Benutzer erkennbar ist. Der Spannungsindikator 101 ist vorzugsweise in der Nähe des verschlussseitigen Bandabschnitts 120 angeordnet, so dass er in jedem Falle der durch die Schlaufe an dem angelegten Körperteil ausgeübten Spannkraft unterliegt.

In dem oberen Teil der Fig. 1 ist die Abschnürvorrichtung 1 im nicht gespannten Zustand des Abschnürbands 10 in Seitenansicht und Draufsicht sowie in einer vergrößerten Ansicht A im Bereich des Spannungsindikators 101 dargestellt. Darunter ist die Abschnürvorrichtung in einem gespannten Zustand des Abschnürbands 10 ebenfalls mit einem vergrößerten Ausschnitt B im Bereich des Spannungsindikators 101 wiedergegeben, wobei das Abschnürband 10 im Bereich eines Dehnabschnitts 100, in dem ein elastisches Element 20 angeordnet ist, um eine definierte Weglänge gedehnt ist. Sobald diese definierte Wegstrecke ausgehend von dem ungespannten Zustand des Abschnürbandes 10 bzw. einem weniger gespannten Zustand erreicht ist, liegt ein definierter Spannungszustand des Abschnürbandes 10 vor. Die dabei bewirkte Spannkraft kann durch die Ausbildung des Dehnabschnitts 100, z. B. durch eine betreffende Materialwahl (Materialeigenschaften und/oder geometrische Eigenschaften, wie Länge, Dicke, Breite, Aussparungen oder dgl.) bei der Fertigung definiert eingestellt werden.

Wie die Fig. 1 weiter zeigt, wird die geschlossene Schlaufe dadurch gebildet, dass das Abschnürband 10 mit seinem von dem verschlussseitigen Bandabschnitt 120 abliegenden endseitigen Bandabschnitt 110 durch eine Verschlussöffnung 121 in dem verschlussseitigen Bandabschnitt 120 bzw. der Verschlussvorrichtung 30 soweit durchgezogen wird, dass die Schlaufe unter der gewünschten Spannung um das abzuschnürende Körperteil geführt ist. Anschließend wird das Abschnürband 10 an dem im Bereich der Verschlussvorrichtung 30 liegenden, die geschlossene Schlaufe begrenzenden Abschnitt des Abschnürbandes 10 in der Verschlussvorrichtung 30 fixiert, um den definierten Spannungszustand einzuhalten. Hierzu weist die Verschlussvorrichtung 30 in dem verschlussseitigen Bandabschnitt 120 ein im Bereich der Verschlussöffnung 121 angeordnetes Halteteil 122 auf. Dieses ist bei dem gezeigten Ausführungsbeispiel als ein von der Verschlussöffnung 121 ausgehender Klemmschlitz ausgebildet, in den der die Schlaufe begrenzende Bandabschnitt des Abschnürbandes 10 von dem Benutzer klemmend eingezogen wird und entgegen der Spannkraft im gespannten Zustand gehalten ist. Der den Klemmschlitz umgebende Bereich ist dabei z. B. durch Verstärkung des verschlussseitigen Bandabschnitts 120 im Bereich der Verschlussvorrichtung 30 entsprechend verstärkt und das Abschnürband 10 einschnürend ausgebildet, um eine genügende Klemmkraft für die Fixierung zu erzeugen.

Wie Fig. 1 in der Draufsicht des zu einer Schlaufe gelegten Abschnürbandes 10 im Bereich der Verschlussvorrichtung 30 zeigt, ist die Verschlussöffnung 121 zu dem Klemmschlitz hin sich verjüngend und in diesen übergehend ausgeführt, so dass das durch die Verschlussöffnung 121 durchgeführte Abschnürband in dem betreffenden Bandabschnitt von dem Benutzer auf einfache Weise in den Klemmschlitz eingezogen und dort fixiert werden kann. Das Abschnürband 10 ist entsprechend flexibel aus einem geschmeidigen, wie z. B. papierähnlichen Material, gegebenenfalls mit Verstärkungsfasern oder Kunststoff ausgebildet und die Klemmeigenschaften des Klemmschlitzes sind auf das Material und die Ausführung des Abschnürbandes 10 abgestimmt, wie z. B. durch Breite des Klemmschlitzes, Griffigkeit und/oder Klemmkraft, so dass das Band sicher fixiert wird.

Zum Abnehmen der Abschnürvorrichtung 1 von dem Körperteil kann das Abschnürband einfach durch Zurückziehen des betreffenden Bandabschnitts entgegen der Einziehrichtung in die Verschlussöffnung 121 zurückgezogen werden, so dass die Klemmkraft aufgehoben wird und die Schlaufe unter Verringerung der Spannkraft gelöst oder geöffnet wird und von dem Körperteil einfach abgenommen werden kann.

In dem gezeigten Ausführungsbeispiel ist das Abschnürband 10 selbst aus inelastischem, aber flexiblem Material hergestellt. Es kann bei der Fertigung der Abschnürvorrichtung 1 als Rollenware vorgehalten und entsprechend der gewünschten Länge des Abschnürbandes 10 abgelängt werden. Vorteilhaft ist ein recyclefähiges Material, wobei auch z. B. natürliche Verstärkungsfasern aus nachwachsenden Rohstoffen eingebunden und auch die übrigen Teile, wie Verschlussvorrichtung, Dehnabschnitt, Befestigungsmittel und dgl. unter Berücksichtigung einer guten Recyclefähigkeit ausgewählt werden können. Die Herstellung, Funktionsweise und die Gestaltung der Abschnürvorrichtung 1 sind vorteilhaft auch hinsichtlich einer nur einmaligen Verwendung auslegbar.

Der Spannungsindikator 101 ist bei dem in Fig. 1 gezeigten Ausführungsbeispiel als Teil des inelastischen Abschnürbandes 10 selbst ausgebildet, indem es in Überlänge an zwei in Dehnrichtung voneinander beabstandeten Fixierstellen an dem aus elastischem Material bestehenden Dehnabschnitt 100 angebunden ist. Zwischen den beiden Fixierstellen verläuft das dort den Spannungsindikator 101 bildende Abschnürband 10 faltenförmig, wobei die Überlänge auf den gewünschten, zu erzeugenden definierten Spannungszustand auch in Abhängigkeit von der Ausbildung des Dehnabschnitts 100 abgestimmt ist. Die Anbindung an den Fixierstellen erfolgt genügend fest z. B. durch Kleben, Schweißen (Laserschweißen, Ultraschweißen oder dgl.), thermoplastische Verbindungsverfahren, Annähen oder dgl.. Beim Spannen des Abschnürbandes 10 kann der Benutzer anhand der sich mehr und mehr dem Dehnabschnitt 100 annähernden Falte den Dehnvorgang und damit die sich erhöhende Spannkraft kontrollieren und eindeutig erkennen, wenn der die beiden Fixierstellen überbrückende Bahnabschnitt des Abschnürbandes 10 seine maximale Strecklage erreicht hat. In diesem Moment ist auch der eingestellte, definierte Spannungszustand bzw. die dabei erzeugte optimale Spannkraft des Abschnürbands 10 erreicht. Soll z. B. der venöse Blutfluss im Bereich der Kapillaren im Übergangsbereich zwischen den Arteriolen und Venolen unterbunden werden, reicht ein dementsprechend eingestellter, definierter Spannungszustand von ca. 10 mm Hg, wobei die zu behandelnde Person wenig belastet wird. Im Bereich von Venen kann der Blutfluss bzw. die Pulsation z. B. durch einen Spannungszustand des Abschnürbandes 10 entsprechend einem Druck von kleiner als 25 mm Hg erreicht werden. Hierfür oder für andere Anwendungen können mit den vorstehend genannten Maßnahmen entsprechend ausgebildete Abschnürvorrichtungen 1 zur Verfügung gestellt werden, wobei der Spannungsindikator 101 und der Dehnabschnitt 100 exakt auf den jeweiligen Anwendungsfall abgestimmt sind.

In entsprechender Weise können auch mehr als ein Spannungsindikator vorzugsweise mit unterschiedlich definierten Spannungszuständen angebracht werden.

Wie Fig. 1 weiter erkennen lässt, ist der endseitige Bandabschnitt 110 zum einfachen Einführen und Durchführen durch die Verschlussöffnung 121 verjüngt, z. B. abgerundet.

Fig. 2A und 2B zeigen eine alternative Ausführung der Abschnürvorrichtung 1, bei der gegenüber dem Ausführungsbeispiel nach Fig. 1 die Verschlussvorrichtung 30 modifiziert ist. Hierbei ist die Verschlussöffnung 121 als quer zu der Längserstreckung bzw. Schlaufe des Abschnürbandes 10 verlaufende schmale, im Wesentlichen rechteckförmige Öffnung ausgeführt, wobei das Abschnürband 10 mit einem verbreiterten Abschnitt im Bereich der Verschlussvorrichtung 30 bzw. des verschlussseitigen Bandabschnitts versehen ist. Hierdurch ist die Ausdehnung der Verschlussöffnung 121 mindestens so breit wie das Abschnürband 10 in seinem über seinen größten Längenbereich einschließlich des endseitigen Bandabschnitts 110 ausführbar bzw. ausgeführt, so dass der endseitige Bandabschnitt 110 und der anschließende Abschnitt des Abschnürbands 10 auch bei stärkerer Ausführung desselben, die kaum eine Formänderung in Querrichtung zulässt, durch die Verschlussöffnung 121 durchführbar ist. Zum Fixieren in dem gewünschten Spannungszustand ist die Verschlussvorrichtung 30 nahe dem verschlussseitigen Bandabschnitt 120 zur Schlaufe hin auf seiner von dem aufzunehmenden Körperteil abgewandten Außenseite mit einem Halteteil 122 versehen, das vorliegend als Klebeteil mit einem Klebeabschnitt ausgeführt ist, der vorzugsweise mit einer leicht entfernbaren oder auf andere Art freigebbaren Abdeckung versehen ist wie z. B. einem durch Faltung aufeinander geklebten Abschnitt gemäß Fig. 2B. Die Kleberschicht kann dabei z. B. auf der Innenseite des endseitigen Bandabschnitts 110 aufgebracht sein. In Fig. 2B ist ein Klebeteil 31 gezeigt.

Der Spannungsindikator 101 ist bei den Ausführungsbeispielen nach Fig. 2A und 2B wiederum in entsprechender Weise wie gemäß Fig. 1 auf der Außenseite der Schlaufe ausgebildet und als Bahnabschnitt mit definierter Überlänge zwischen zwei Fixierstellen ausgeführt, an denen das elastische Element 20 als Dehnabschnitt angebunden ist. In Fig. 2 ist in der Mitte oben der nicht gespannte und in der rechten Darstellung die Abschnürvorrichtung mit dem Abschnürband 10 in dem definierten Spannungszustand gezeigt.

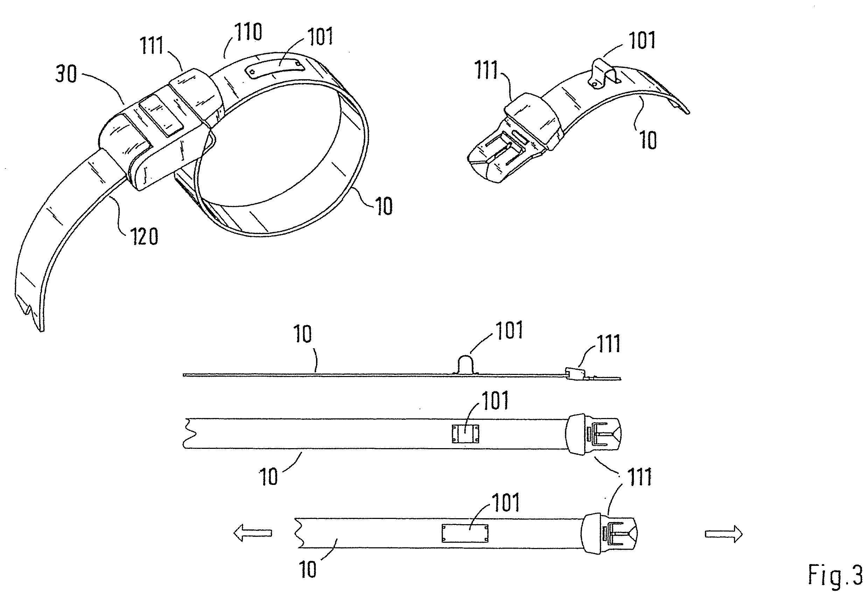

Fig. 3 zeigt ein anderes Ausführungsbeispiel der Abschnürvorrichtung 1. Hierbei ist das Abschnürband 10 selbst aus elastischem Material ausgeführt. Der daran an den in Dehnungsrichtung voneinander beabstandeten Fixierstellen angebundene Spannungsindikator 101 mit definierter Überlänge besteht aus flexiblem unelastischem Material, wie bei dem vorhergehenden Ausführungsbeispiel. Wird das Abschnürband 10 gedehnt, legt sich der gefaltete Bahnabschnitt des Spannungsindikators 101 mehr und mehr an die außenseitige Oberfläche des Abschnürbandes 10 an. Dabei ist die Überlänge des Bahnabschnittes auf den überbrückten Abschnitt des elastischen Abschnürbands abgestimmt, der dann dem elastischen Element 20 nach den vorherigen Ausführungsbeispielen entspricht, und zwar in der Weise, dass bei vollständiger Erstreckung des Bahnabschnitts entlang dem betreffenden Abschnitt des Abschnürbands 10 ohne Falte der gewünschte definierte Spannungszustand erreicht ist. In diesem Zustand erfolgt dann die Fixierung der Schlaufe durch den Benutzer, ohne das Abschnürband noch stärker zu spannen. Bei dem gezeigten Ausführungsbeispiel ist die Verschlussvorrichtung mit einem Schlossgehäuse und einer darin gelagerten Klemmwippe z. B. entsprechend der bereits genannten EP 1 458 296 B1 oder der EP 0 633 747 B1 ausgebildet, wobei der verschlussseitige Bandabschnitt 110 mit einem in dem Schlossgehäuse freigebbar gekoppelten Rastschuh versehen ist. Über die gelöste Klemmwippe kann die Größe und Spannung der Schlaufe von dem Benutzer dosiert verstellt werden.

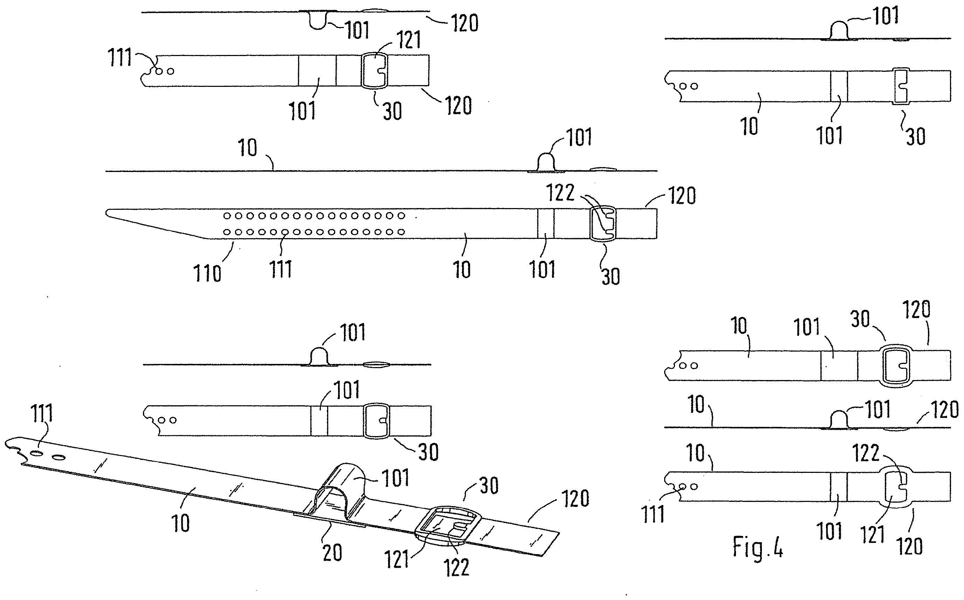

Bei den in Fig. 4 gezeigten weiteren Ausführungsbeispielen der Abschnürvorrichtung ist der Spannungsindikator 101 wiederum entsprechend den Ausführungsbeispielen nach den Fig. 1 und 2 mit einem elastischen Element 20 ausgeführt, das an dem aus einem unelastischen flexiblen Material gebildeten Abschnürband 10 angebracht ist. Die Verschlussvorrichtung 30 ist jedoch gegenüber den vorhergehenden Ausführungsbeispielen modifiziert. Hierbei ist in dem verschlussseitigen Bandabschnitt 120 im Bereich der Verschlussvorrichtung eine Verschlussöffnung 121 gebildet, die der Breite des durchgeführten Abschnitts des Abschnürbands 10 im Wesentlichen entspricht, wozu der betreffende Verschlussteil nach außen hin verbreitert ist. Das Halteteil 122 ist als zapfenartiger Vorsprung ausgebildet, der in die Verschlussöffnung 121 ragt, während als Koppelglied 111 mit dem zapfenartigen Halteteil 122 im Spannungszustand der Schlaufe in Eingriff bringbare Löcher in dem betreffenden Abschnitt des Abschnürbandes 10 eingebracht sind. Diese Haltelöcher sind in Form einer oder mehrerer, z. B. zwei nebeneinander liegender, in Dehnrichtung verlaufender Lochreihen vorzugsweise in regelmäßigem Rasterabstand angeordnet. Auf diese Weise lässt sich der Schlaufendurchmesser und die erzeugte Spannkraft variieren, um den definierten Spannungszustand möglichst genau zu erreichen.

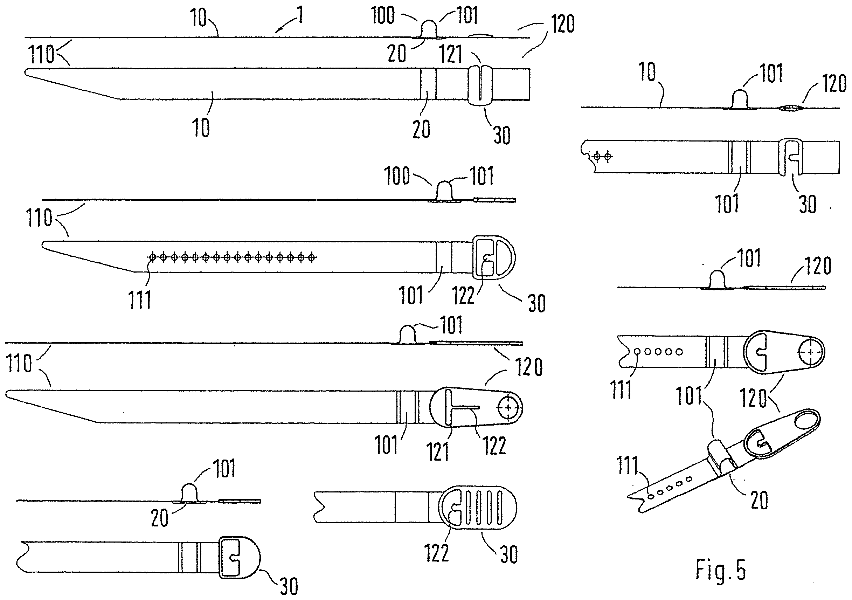

Bei den in Fig. 5 weiterhin gezeigten Ausführungsbeispielen sind noch andere Verschlussvorrichtungen 30 vorgesehen, nämlich ein zu einer längsseitig in offener, quer verlaufender Schlitz, in den der betreffende Abschnitt des Abschnürbandes 10 zum Bilden der Schlaufe seitlich einsetzbar ist und der eine genügende Klemmkraft für die Fixierung des Abschnürbandes 10 bei dem eingestellten Spannungszustand ergibt, insbesondere in dem definierten Spannungszustand. Die weiteren Ausführungsformen der Verschlussvorrichtung 30 enthalten wiederum eine Verschlussöffnung 121 mit einem als zapfenförmiger Vorsprung in diese hineinragenden Halteteil 122, einen in Längsrichtung bzw. Dehnrichtung des Abschnürbandes 10 verlaufende Längsschlitz als Halteteil 122, der in die quer verlaufende Verschlussöffnung 121 übergeht, bzw. an dem verschlussseitigen Bandabschnitt 120 angeformte Abschnitte, die weiterhin noch mit einer endseitigen Öffnung zum Aufbewahren der Abschnürvorrichtung 1 an einem Haken versehen sind. Die endseitigen Bandabschnitte 110 sind jeweils sich verjüngend ausgeführt, um sie problemlos in die Verschlussöffnung 121 einführen zu können.

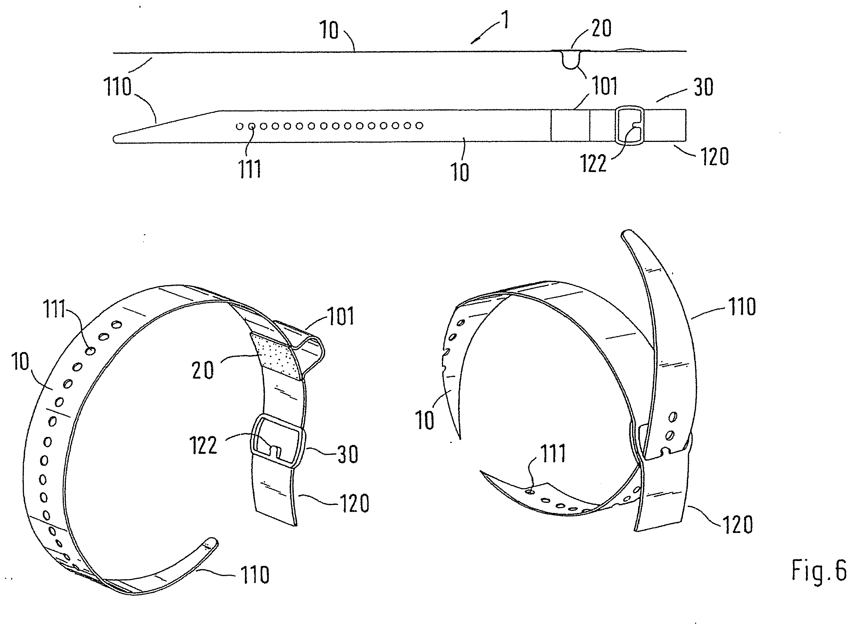

Fig. 6 zeigt wiederum eine Ausführungsform der Abschnürvorrichtung 1, bei der die Verschlussvorrichtung 30 eine Verschlussöffnung 121 mit einem in diese ragenden zapfenartigen Vorsprung als Halteteil 122 aufweist. Zum Bilden und Fixieren der Schlaufe weist das Abschnürband 10 in betreffendem Abstand von der Verschlussöffnung 121 eine in Dehnrichtung verlaufende Lochreihe mit Löchern als Koppelglieder 111 auf, in die der als Halteteil 122 dienende zapfenartige Vorsprung unter betreffender Zugspannung einsetzbar ist.

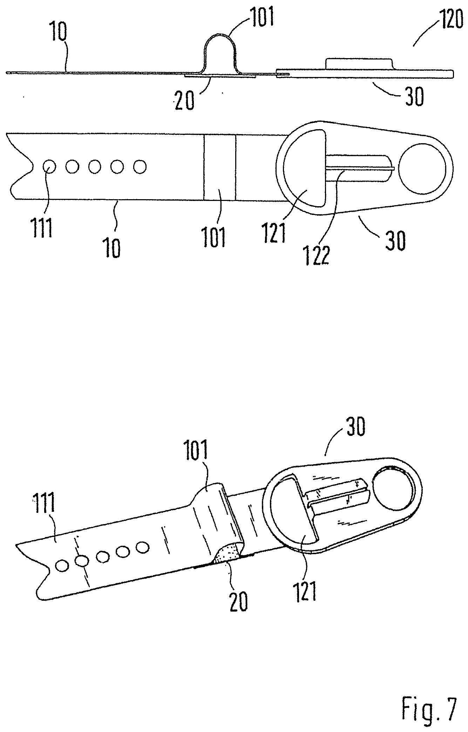

Bei der in Fig. 7 gezeigten Ausführung ist wiederum eine Verschlussöffnung 121 mit einem als Halteteil 122 dienenden Klemmschlitz vorhanden, wobei der Klemmschlitz mit verstärkten Flanken für eine sichere Klemmung und Fixierung des zu einer Schlaufe gelegten Abschnürbandes 10 ausgebildet ist. Der betreffende Abschnitt der Verschlussvorrichtung 30 ist beispielsweise als separater Kunststoffabschnitt ausgeführt, der mit dem verschlussseitigen Bandabschnitt 120 stabil verbunden ist, wie z. B. durch Schweißen, Anformen, Kleben oder Klemmen. Gezeigt ist auch der Spannungsindikator 101.

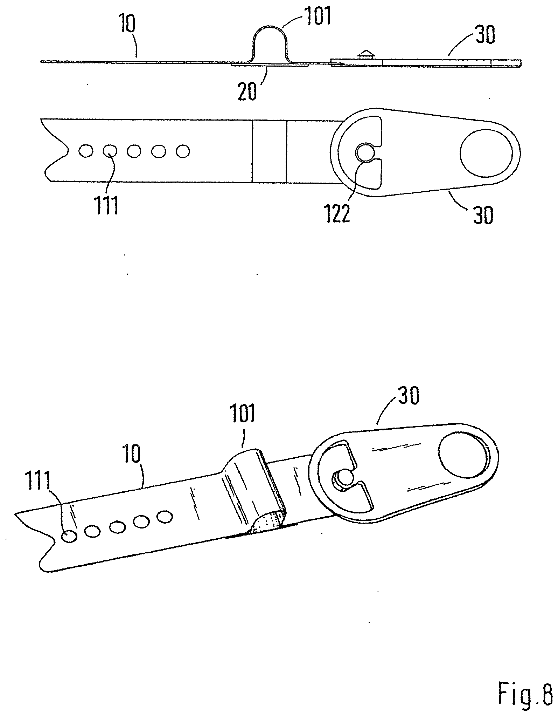

Eine weitere Ausführungsform der Verschlussvorrichtung ist in Fig. 8 dargestellt, wobei der zapfenartige Vorsprung eine gegenüber der Oberseite des Abschnürbands 10 vorspringende Einhängenase aufweist, in die als Löcher ausgebildete Koppelglieder 111 in dem betreffenden Abschnitt des Abschnürbandes 10 bei geeigneter Zugspannung, insbesondere also unter dem definierten Spannungszustand in Eingriff bringbar sind. Auch hierbei ist der nahe dem verschlussseitigen Bandabschnitt 120 angeordnete Spannungsindikator 101 dargestellt.

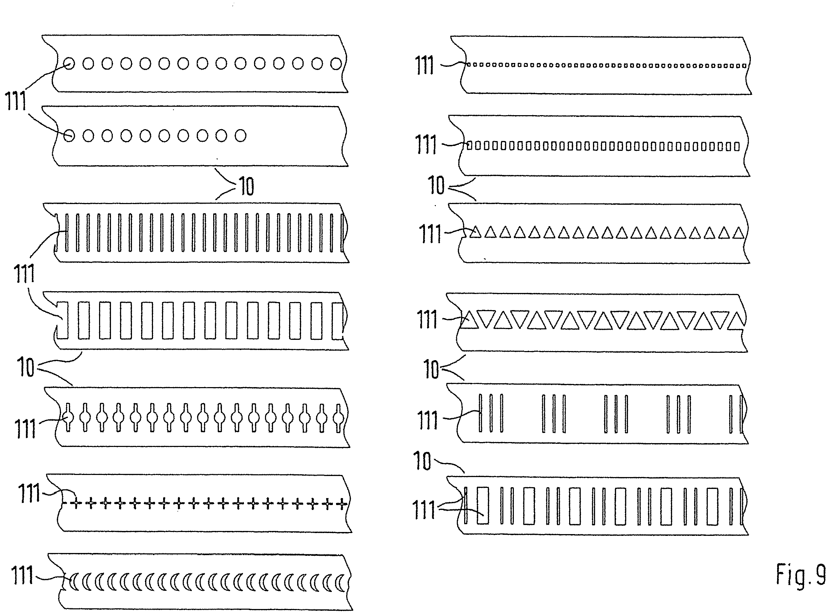

Fig. 9 zeigt verschiedene Lochfigurationen als Koppelglieder 111, die mit entsprechend ausgebildeten (nicht gezeigten) zapfenartigen Vorsprüngen in dem gegenüberliegenden Verschlussabschnitt der Verschlussvorrichtung 30 in Eingriff bringbar sind.

Als Verschlussvorrichtung 30 ist auch ein Klettverschluss geeignet, der lediglich aus zwei miteinander in Eingriff bringbaren Klettverschlussteilen gebildet ist, wie z. B. ein flanschiges Band als Abschnürband 10 einerseits und ein damit zusammen wirkender, auf dem Band befestigter Abschnitt mit Kletthäkchen andererseits.

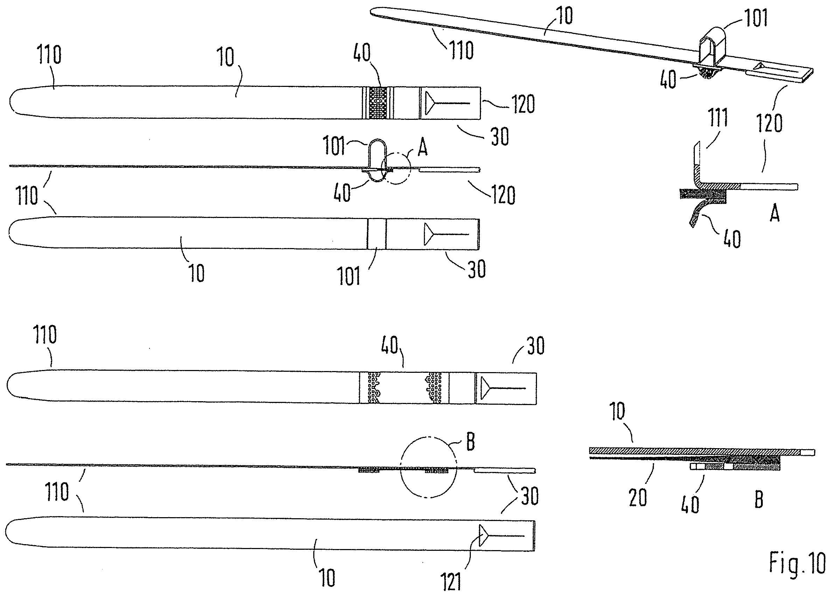

Eine weitere Ausgestaltung der Abschnürvorrichtung 1 ist in Fig. 10 gezeigt. Hierbei ist die Abschnürvorrichtung entsprechend dem Ausführungsbeispiel nach Fig. 1 mit einem Spannungsindikator 101 versehen, der als Bahnabschnitt in Überlänge bezüglich des unbelasteten Abschnürbandes ein elastisches Element 20 zwischen zwei Fixierstellen überbrückt, wie vorstehend näher beschrieben. Auch die Verschlussvorrichtung 30 ist entsprechend dem Ausführungsbeispiel nach Fig. 1 ausgebildet. Zusätzlich ist jedoch ein Gebrauchsindikator 40 vorhanden, der bei dem gezeigten Ausführungsbeispiel ebenfalls das elastische Element 20 oder zumindest einen in Längsrichtung erstreckten Abschnitt desselben überbrückt.

Der Gebrauchsindikator 40 ist als in Längsrichtung bzw. Dehnrichtung sich erstreckendes Durchreißelement ausgebildet, welches bei Dehnung des elastischen Elements 20 mit geringem Kraftaufwand reißt, bevor die endgültige Dehnung erreicht ist, die bei dem definierten Spannungszustand vorliegt. Ähnlich dem Durchreißelement kann auch ein anderes irreversibel zu schädigendes Element, wie z. B. ein Lack, angebracht werden. In welchem Bereich während des Dehnvorgangs das Durchreißelement reißen soll, kann ebenfalls durch eine gewisse Überlänge zwischen zwei Fixierungsstellen bzw. Befestigungsstellen des Durchreißelements an dem elastischen Element 20 bestimmt werden, wobei die Überlänge des Durchreißelements aber jedenfalls geringer ist als die Überlänge des für den Spannungsindikator 101 vorgesehenen Bahnabschnitts. Beispielsweise kann auf diese Weise bestimmt werden, dass das Durchreißelement bereits durchreißt, wenn die Dehnung des elastischen Elements z. B. ca. 20 %, ca. 50 % oder ca. 80 % oder einen anderen, geringeren Zwischenwert relativ zu der bei dem definierten Spannungszustand vorliegenden Dehnung erreicht hat. Beliebige Zwischenwerte für ein Durchreißen können auf diese Weise exakt eingestellt werden. Ist das Durchreißelement des Gebrauchsindikators 40 gerissen, bedeutet dies, dass die Abschnürvorrichtung 1 bereits in Gebrauch war und somit keine Erstverwendung vorliegt, wie es bei Abschnürvorrichtungen 1 für den Einmalgebrauch erforderlich ist. Das Durchreißelement kann z. B. aus einem leicht reißenden Papierstreifen oder Faserstreifen bestehen und ist vorzugsweise inelastisch, jedoch flexibel.

Sind die Fixierstellen des Spannungsindikators 101 und die Befestigungsstellen des Gebrauchsindikators 40 in Draufsicht deckungsgleich, so können beide Indikatoren einfach in einem gemeinsamen Arbeitsschritt hergestellt und auch als Funktionseinheit aufeinander exakt in gewünschter Weise abgestimmt werden.

Die Anbindung des für den Gebrauchsindikator 40 verwendeten Durchreißelements an den betreffenden Fixierstellen bzw. Befestigungsstellen kann in entsprechender Weise erfolgen, wie im Zusammenhang mit dem elastischen Element 20 beschrieben, also beispielsweise durch Kleben, Schweißen (Laserschweißen, Ultraschall, Aufdruck mittels Drucktechnik oder dgl.), Vernähen oder eine thermoplastische Verbindungstechnik oder aber eine andere geeignete Befestigungsart. In der vergrößerten Darstellung des Ausschnitts A ist ein Verbindungsabschnitt (Befestigungsstelle) gezeigt, wobei das elastische Element 20 auf seiner Oberseite an dem Abschnürband 10 fixiert ist und auf seiner Unterseite mit dem betreffenden Befestigungsabschnitt des Durchreißelements verbunden ist.

In der unteren Bildhälfte der Fig. 10 ist der gedehnte Zustand des Abschnürbands 10 gezeigt, wie er beispielsweise in dem definierten Spannungszustand vorliegt. Hierbei ist das Durchreißelement des Gebrauchsindikators 40 durch die Dehnung bereits gerissen, wobei die beiden rissseitigen Ränder um einen relativ großen Dehnspalt voneinander beabstandet sind, d. h. der Durchriss bereits in einem entsprechend frühen Stadium der Dehnung erfolgt ist. In der vergrößerten Darstellung des Durchrissbereichs (Detail B) sind der eine (verschlussseitige) verbleibende Abschnitt des Durchreißelements, das bei der Dehnung dünner werdende elastische Element 20 sowie der gestreckte Bahnabschnitt des Spannungsindikators 101 zu sehen.

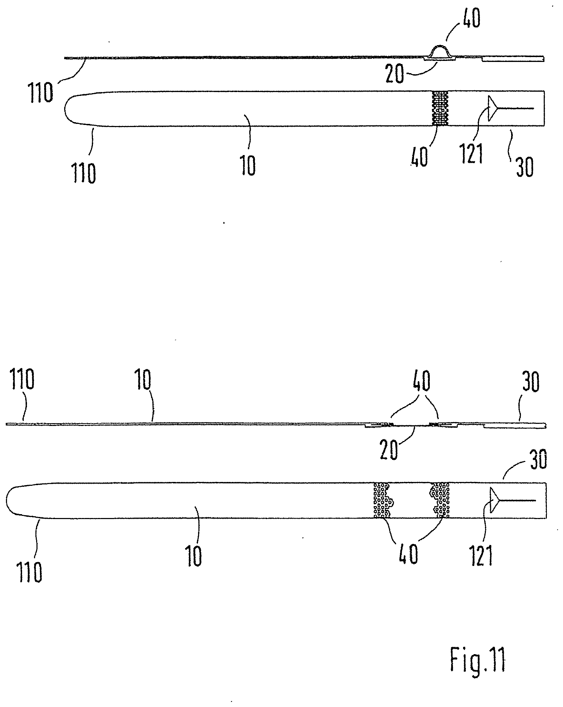

Wie Fig. 11 zeigt, kann die Abschnürvorrichtung 1 auch unabhängig von einem Spannungsindikator 101 oder davon in Längsrichtung versetzt einen Gebrauchsindikator 40 an dem Abschnürband 10 aufweisen, wobei letzterer ein elastisches Element 20 zwischen zwei Fixierstellen bzw. Befestigungsstellen mit (oder alternativ ohne) Überlänge überbrückt, ähnlich wie bei der Ausführung nach Fig. 10 beschrieben. Das Abschnürband 10 kann außerhalb des elastischen Elements 20 wiederum aus inelastischem (= unelastischem), flexiblem Material hergestellt sein.

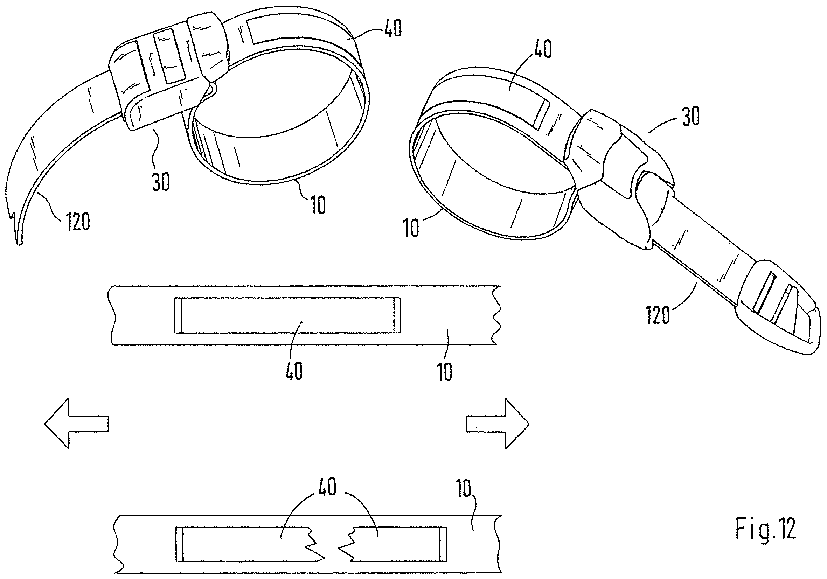

Bei dem in Fig. 12 gezeigten Ausführungsbeispiel ist der Gebrauchsindikator 40 mit einem Durchreißelement auf einem elastischen Abschnürband 10 aufgebracht, wobei keine Überlänge des Durchreißelements vorgesehen sein muss, wie dies auch bei den Ausführungsbeispielen nach Fig. 10 und Fig. 11 der Fall sein könnte. Auch hierbei besitzt das Durchreißelement eine geringe Durchreißkraft, so dass es bei einer ersten Dehnung reißt und anzeigt, dass die Abschnürvorrichtung 1 bereits gebraucht wurde. Die Verschlussvorrichtung 121 kann z. B. entsprechend den genannten Druckschriften EP 0 633 747 B1 bzw. EP 1 458 296 B1 ausgebildet sein.

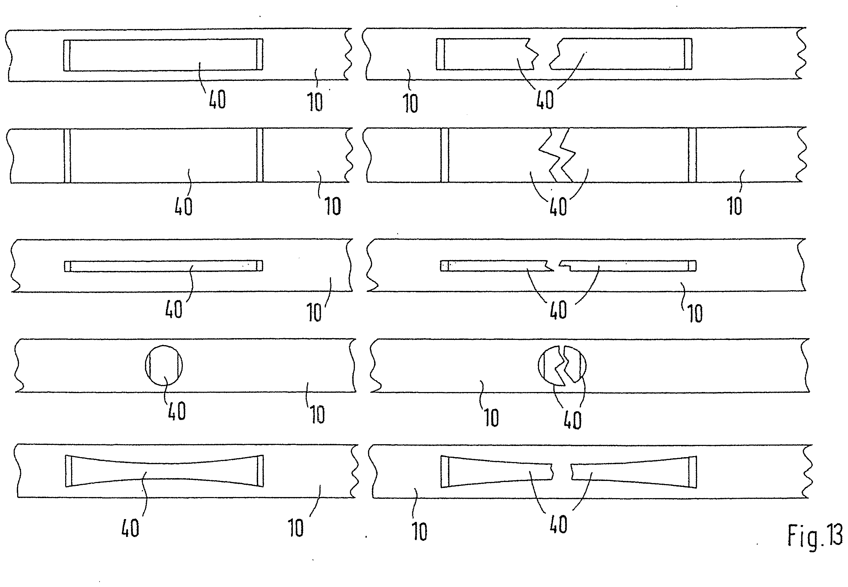

In Fig. 13 sind verschiedene Ausgestaltungsvarianten von Gebrauchsindikatoren 40 in Verbindung mit Abschnürbändern 10 beispielhaft dargestellt. Während in der linken Bildhälfte jeweils ein ungedehnter Zustand gezeigt ist, ist in der rechten Bildhälfte ein gedehnter Zustand mit durchgerissenem Durchreißelement dargestellt. Ferner sind die an den beiden Endabschnitten des Durchreißelements vorgesehenen Fixierstellen wiedergegeben. Durch geeignete Materialwahl bzw. Wahl der Geometrie insbesondere im Durchreißabschnitt kann die Durchreißkraft und auch der Augenblick bzw. der erforderliche Dehnweg vorgegeben werden, bei dem/der der Durchriss erfolgt.

Fig. 14 zeigt verschiedene Ausgestaltungsvarianten für Abschnürvorrichtungen 1 mit unterschiedlichen endseitigen Bandabschnitten 110, die für eine einfache Einführung in die Verschlussöffnung 121 vorgesehen sein können.

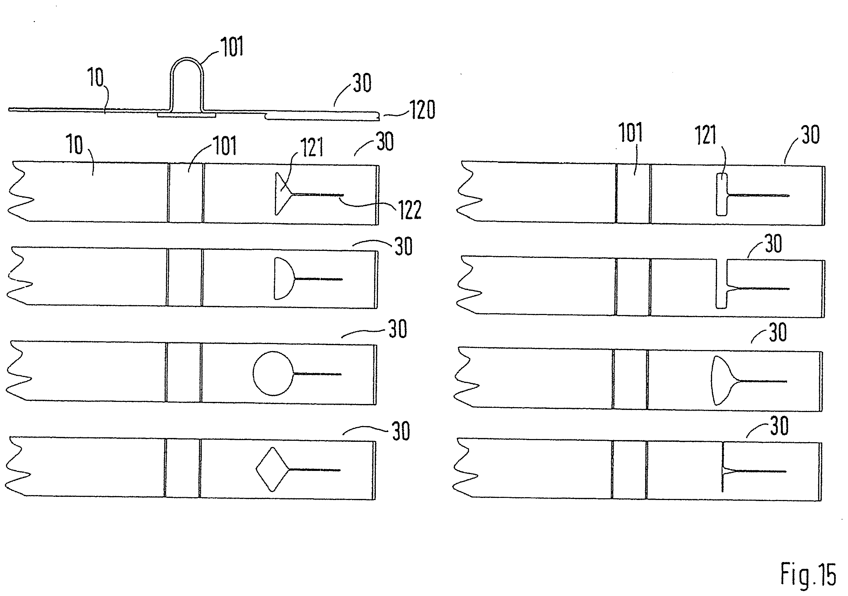

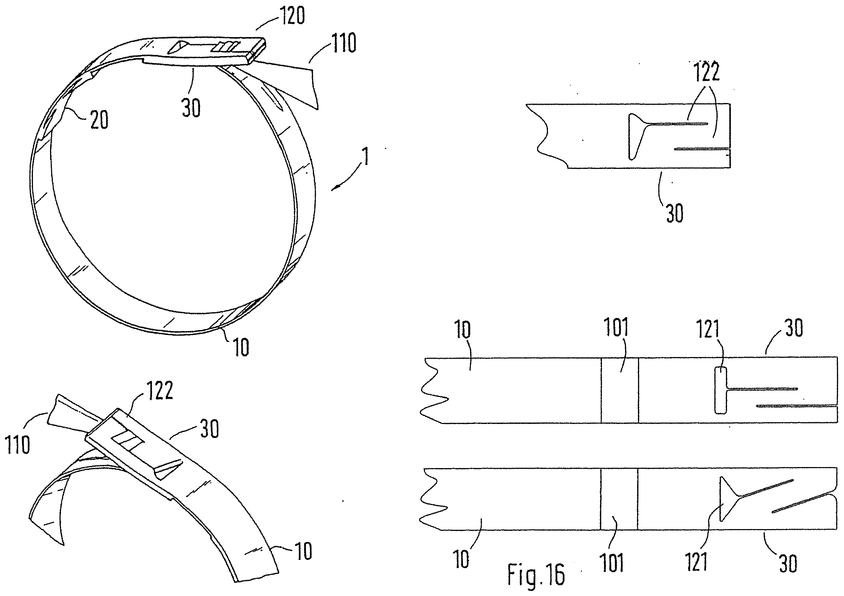

In Fig. 15 sind verschiedene Ausgestaltungsvarianten von Verschlussvorrichtungen 30 mit verschiedenen Formen von Verschlussöffnungen 121 und als Klemmschlitz ausgebildeten Halteteilen 122 dargestellt. Weitere Darstellungen der Verschlussvorrichtung 30 sind in Fig. 16 gezeigt. Hierbei kann insbesondere bei dünnem, sehr flexiblem Material des Abschnürbandes 10 eine Anordnung von zwei nebeneinander liegenden Klemmschlitzen vorgesehen sein, von denen der eine in die Verschlussöffnung 121 übergeht und der andere an dem schmalen Ende des verschlussseitigen Bandabschnitts 120 geöffnet ist, so dass der betreffende, die Schlaufe begrenzende Bandabschnitt in dem an die Verschlussöffnung 121 angrenzenden Klemmschlitz zunächst einfach und schnell vorfixiert und anschließend in den zweiten Klemmschlitz vom schmalen Ende her eingeführt und stabil festgelegt werden kann, um den definierten Spannungszustand zuverlässig zu sichern. Auch bei dieser Ausführungsform ist ein Spannungsindikator 101 mit einem elastischen Element 20 vorhanden. Ferner kann auch hierbei ein Gebrauchsindikator 40 mit dem vorstehend beschriebenen Aufbau vorgesehen sein.

## Patentansprüche

1. Venenstauer, mit einem um ein Körperteil legbaren Abschnürband (10), einer Verschlussvorrichtung (30), mittels deren das Abschnürband (10) in seinem um ein Körperteil gelegten gespannten Zustand zu einer Schlaufe verriegelbar ist, und mit mindestens einem Spannungsindikator (101) zum Feststellen eines Spannungszustandes des Abschnürbandes (10), der als ein einen elastischen Abschnitt am Abschnürband (10) in Dehnrichtung zwischen zwei Fixierstellen mit Überlänge bezüglich des ungedehnten Abschnürbandes (10) überbrückender Bahnabschnitt aus einem flexiblen unelastischen Material ausgebildet ist, wobei der Spannungsindikator (101) in der Weise ausgebildet ist, dass ein herstellerseitig vorgegebener definierter Spannungszustand, der auf den Anwendungsfall als Venenstauer entsprechend einem Druck von kleiner als 25 mm Hg abgestimmt ist, von einem Benutzer erkennbar ist, wobei der Spannungsindikator (101) als ein den elastischen Abschnitt am Abschnürband (10) in Dehnrichtung zwischen den zwei Fixierstellen mit definierter Überlänge bezüglich des unbelasteten Abschnürbandes (10) überbrückender Bahnabschnitt aus dem flexiblen unelastischen Material ausgebildet ist und wobei der definierte Spannungszustand in dem Moment erreicht ist, wenn der die beiden Fixierstellen überbrückende Bahnabschnitt des Abschnürbandes (10) beim Dehnvorgang seine maximale Strecklage erreicht hat ohne das Abschnürband noch stärker zu spannen und
wobei der Spannungsindikator (101) auf der im Gebrauchszustand von dem Körperteil abgekehrten Außenseite des Abschnürbandes (10) angeordnet ist.

2. Venenstauer nach Anspruch 1,
**dadurch gekennzeichnet,**
**dass** das Abschnürband (10) aus einem flexiblen unelastischen Material ausgebildet ist und der Bahnabschnitt einen integralen Teil des Abschnürbandes (10) bildet, wobei an den Fixierstellen ein den elastischen Abschnitt bildendes elastisches Element (20) angebunden ist.

3. Venenstauer nach Anspruch 1 oder 2,
**dadurch gekennzeichnet,**
**dass** das Abschnürband (10) selbst zumindest abschnittsweise als elastisches Band ausgebildet ist und der Bahnabschnitt aus unelastischem Material an den Fixierstellen an dem elastischen Band angebunden ist.

4. Venenstauer nach einem der vorhergehenden Ansprüche,
**dadurch gekennzeichnet,**
**dass** der elastische Abschnitt oder ein anderer Abschnitt des elastischen Bandes mit einem bei einer ersten Dehnung durchreißenden Gebrauchsindikator (40) versehen ist.

5. Venenstauer nach einem der vorhergehenden Ansprüche,
**dadurch gekennzeichnet,**
**dass** ein Endabschnitt des Abschnürbandes (10) als verschlussseitiger Bandabschnitt mit einer Verschlussöffnung (121) zum Durchführen des anderen endseitigen Bandabschnitts (110) und mit einem Halteteil (122) zum Festhalten des um einen Körperteil zu einer Schlaufe gelegten Abschnürbandes (10) im gespannten Zustand versehen ist.

6. Venenstauer nach Anspruch 5,
**dadurch gekennzeichnet,**
**dass** das Halteteil (122) als mindestens ein Klemmschlitz, als in eine Lochanordnung des Abschnürbandes (10) eingreifender Haltezapfen, als Klettverschlussabschnitt, als Klebeteil, als Druckknopfteil oder als schwenkbare Klemmplatte ausgebildet ist.

7. Venenstauer nach einem der vorhergehenden Ansprüche,
**dadurch gekennzeichnet,**
**dass** die Verschlussvorrichtung (30) durch einen Klettverschluss gebildet ist, der lediglich aus zwei miteinander in Eingriff bringbaren Klettverschlussteilen gebildet ist, wobei einerseits als Abschnürband (10) ein flauschiges Band und andererseits ein damit zusammenwirkender, auf dem Band befestigter Abschnitt mit Kletthäkchen vorhanden sind.

8. Venenstauer nach einem der vorhergehenden Ansprüche,
**dadurch gekennzeichnet,**
**dass** das Abschnürband (10) aus reißfestem gewebeartigen, vliesartigen oder papierartigen Material hergestellt ist.

9. Venenstauer nach einem der Ansprüche 2 bis 8,
**dadurch gekennzeichnet,**
**dass** die Anbindung des elastischen Abschnitts nach Anspruch 2 oder des Bahnabschnitts an dem Abschnürband (10) an den Fixierstellen mittels Klebens, Schweißens, thermoplastischer Verfahren oder Vernähens hergestellt ist.

## Claims

1. Tourniquet comprising a constricting band (10) that can be placed around a body part, a closure device (30) by means of which the constricting band (10) can be locked in position when placed under tension in a loop around a body part, and at least one tension indicator (101) for determining a tension state of the constricting band (10), which indicator is designed as a web portion, made of a flexible non-resilient material, that spans a resilient portion on the constricting band (10) between two fastening points in the stretching direction, adding excess length with respect to the unstretched constricting band (10), wherein the tension indicator (101) is designed such that a tension state, which is predefined by the manufacturer and adjusted for tourniquet use corresponding to a pressure of less than 25 mm Hg, is visible to a user, wherein the tension indicator (101) is designed as a web portion, made of a flexible non-resilient material, that spans the resilient portion on the constricting band (10) between the two fastening points in the stretching direction, adding a defined excess length with respect to the unstretched constricting band (10), and wherein the defined tension state is reached when the web portion of the constricting band (10) which spans the two fastening points has reached its maximum extended position during the stretching process without further tensioning the constricting band, and wherein the tension indicator (101) is arranged on the outer face of the constricting band (10) which faces away from the body part when in use.

2. Tourniquet according to claim 1, **characterized in that** the constricting band (10) is made of a flexible non-resilient material and the web portion forms an integral part of the constricting band (10), a resilient element (20) that forms the resilient portion being connected to the fastening points.

3. Tourniquet according to either claim 1 or claim 2, **characterized in that** the constricting band (10) itself is designed as a resilient band, at least in part, and the web portion made of non-resilient material is connected to the fastening points on the resilient band.

4. Tourniquet according to any of the preceding claims, **characterized in that** the resilient portion or another portion of the resilient band is provided with a usage indicator (40) that tears when first stretched.

5. Tourniquet according to any of the preceding claims, **characterized in that** an end portion of the constricting band (10) is provided, as a closure-side band portion, with a closure opening (121) through which the other end-side band portion (110) can pass, and a retaining part (122) for securing, in a tensioned state, the constricting band (10) that forms a loop around a body part.

6. Tourniquet according to claim 5, **characterized in that** the retaining part (122) is designed as a clamping slot, as a retaining catch that engages in a hole assembly of the constricting band (10), as a hook-and-loop fastener portion, as an adhesive part, as a snap fastener portion or as a pivotable clamping plate.

7. Tourniquet according to any of the preceding claims, **characterized in that** the closure device (30) is formed of a hook-and-loop fastener merely consisting of two hook-and-loop parts that can be brought into mutual engagement, a fleece band forming the constricting band (10), and a portion interacting therewith that is fastened to the band and comprises hooks being provided.

8. Tourniquet according to any of the preceding claims, **characterized in that** the constricting band (10) is produced from tear-resistant woven, nonwoven or paper-like material.

9. Tourniquet according to any of claims 2 to 8, **characterized in that** the connection between the resilient portion according to claim 2, or the web portion on the constricting portion (10), and the fastening points is produced by means of adhesion, welding, a thermoplastic process or stitching.

## Revendications

1. Garrot veineux, comprenant une bande d'étranglement (10) pouvant être placée autour d'une partie du corps, un dispositif de fermeture (30) au moyen duquel la bande d'étranglement (10) peut être verrouillée, lorsqu'elle enroulée autour d'une partie du corps, pour former une boucle, et au moins un indicateur de tension (101), destiné à déterminer un état de tension de la bande d'étranglement (10), et qui est conçu comme une partie de bande, en matière non élastique flexible, qui couvre une partie élastique au niveau de la bande d'étranglement (10) dans la direction d'étirement entre deux points de fixation avec un excès de longueur par rapport à la bande d'étranglement (10) non étirée, l'indicateur de tension (101) étant conçu de manière à ce qu'un état de tension défini, prescrit par le fabriquant, qui est adapté à l'application comme garrot veineux à une pression inférieure à 25 mm Hg, puisse être reconnu par un utilisateur, l'indicateur de tension (101) étant conçu comme une partie de bande, en matière non élastique flexible, qui couvre la partie élastique au niveau de la bande d'étranglement (10) dans la direction d'étirement entre les deux points de fixation avec un excès de longueur défini par rapport à la bande d'étranglement (10) non étirée, et l'état de tension défini étant atteint au moment où la partie de bande, couvrant les deux points de fixation, de la bande d'étranglement (10) a atteint lors du processus d'étirement sa position d'étirement maximale sans serrer la bande d'étranglement encore plus fort et l'indicateur de tension (101) étant disposé du côté extérieur de la bande d'étranglement (10) qui est opposé à la partie du corps pendant l'utilisation.

2. Garrot veineux selon la revendication 1,
**caractérisé en ce que**
la bande d'étranglement (10) est constituée d'une matière non élastique flexible et la partie de bande fait partie intégrante de la bande d'étranglement (10), un élément élastique (20) qui forme la partie élastique étant fixé aux points de fixation.

3. Garrot veineux selon la revendication 1 ou 2,
**caractérisé en ce que**
la bande d'étranglement (10) elle-même est conçue au moins partiellement comme une bande élastique et la partie de bande en matière non élastique est fixée à la bande élastique au niveau des points de fixation.

4. Garrot veineux selon l'une des revendications précédentes,
**caractérisé en ce que**
la partie élastique ou une autre partie de la bande élastique est munie d'un indicateur d'utilisation (40) se déchirant lors d'un premier étirement.

5. Garrot veineux selon l'une des revendications précédentes,
**caractérisé en ce que**
une partie d'extrémité de la bande d'étranglement (10) en tant que partie de bande côté fermeture est pourvue d'une ouverture de verrouillage (121) destinée au passage de l'autre partie de bande côté extrémité (110) et d'un élément de retenue (122) destiné à retenir la bande d'étranglement (10) placée autour d'une partie du corps pour former une boucle à l'état tendu.

6. Garrot veineux selon la revendication 5,
**caractérisé en ce que**
l'élément de retenue (122) est conçu comme au moins une fente de serrage, comme une tige de retenue s'engageant dans un agencement de trous de la bande d'étranglement (10), comme une partie de fermeture auto-agrippante, comme un élément adhésif, comme un élément à bouton-poussoir ou comme une plaque de serrage pivotante.

7. Garrot veineux selon l'une des revendications précédentes,
**caractérisé en ce que**
le dispositif de fermeture (30) est formé par une fermeture auto-agrippante qui est formée seulement de deux éléments de fermeture auto-agrippante pouvant s'engager l'un avec l'autre, d'une part une bande pelucheuse servant de bande d'étranglement (10) et d'autre part une partie, munie de petits crochets d'agrippement, qui est fixée sur la bande et coopère avec la bande pelucheuse, étant prévues.

8. Garrot veineux selon l'une des revendications précédentes,
**caractérisé en ce que**
la bande d'étranglement (10) est réalisée à partir d'une matière résistante à la déchirure, de type tissu, non-tissé ou papier.

9. Garrot veineux selon l'une des revendications 2 à 8,
**caractérisé en ce que**
la fixation de la partie élastique selon la revendication 2 ou de la partie de bande à la bande d'étranglement (10) est réalisée au niveau des points de fixation par collage, soudage, procédé thermoplastique ou couture.
